# EUROPEAN PATENT SPECIFICATION

(11) **EP 3 085 319 B1**
(45) Date of publication and mention of the grant of the patent: **11.10.2017**
(21) Application number: 15164551.2
(22) Date of filing: 21.04.2015
(51) Int. Cl.: A61B 17/70, A61B 17/80

(54) **TEMPLATE FOR USE IN MANUFACTURING AN IMPLANT FOR SPINAL OR OTHER ORTHOPAEDIC FIXATION AND METHOD OF MANUFACTURING SUCH A TEMPLATE**
SCHABLONE ZUR VERWENDUNG BEI DER HERSTELLUNG EINES IMPLANTATS FÜR DIE WIRBELSÄULEN- ODER ANDERE ORTHOPÄDISCHE FIXIERUNG UND VERFAHREN ZUR HERSTELLUNG SOLCH EINER SCHABLONE
GABARIT DESTINÉ À ÊTRE UTILISÉ DANS LA FABRICATION D'UN IMPLANT DE FIXATION ORTHOPÉDIQUES SPINAL OU AUTRES ET PROCÉDÉ DE FABRICATION D'UN TEL GABARIT

(43) Date of publication of application: 26.10.2016
(73) Proprietor: Biedermann Technologies GmbH & Co. KG, 78166 Donaueschingen (DE)
(72) Inventor: Biedermann, Lutz, 78048 VS-Villingen (DE); Zipse, Achim, 76532 Baden-Baden (DE)
(74) Representative: Prüfer & Partner mbB Patentanwälte · Rechtsanwälte

(56) References cited:
- US-A- 4 170 990
- US-A- 5 290 289
- US-A1- 2007 191 831
- US-A1- 2010 063 548

## Description

The invention relates to a template for use in manufacturing an implant for spinal or other orthopaedic fixation and to a method of manufacturing such an implant. The template is capable of bending in response to a force and is capable of retaining a bent shape which is used to model the implant with a shape corresponding to the shape of the template.

It is known in the medical art to use spinal rod templates for modelling the shape of a permanent rod that is used for stabilizing the spine by means of a screw-rod system. The necessary shape and length of the spinal rod which is to be implanted into a patient is determined on the basis of the rod template which is a rod that is initially bent and measured to conform to the effected part of the patient's spine. The rod template is then utilized for the configuring of the permanent rod which is to be implanted into the patient and secured to a portion of the length of the spine. Such templates are, for example, known from US 6,221,077 B1.

The rod templates are bent manually or with a tool in the operating room (OR). To permit bending in the OR, rod templates known in the art can be made of an aluminium alloy, for example AlMgSi, having a relatively low strength. After use in the manufacture of the permanent rod implants, the rod templates are bent back and sterilized for using them a second time. This procedures may be repeated several times. However, the used rod templates may still have a remaining curvature. In addition, there may be a risk of breaking when the rod templates are bent and bent back several times.

US 2010/0063548 A1 describes a spinal correction template having a first configuration which substantially corresponds to an uncorrected shape of a spine. The spinal correction template can be activated such that the template achieves a second configuration to cause the spine to assume an orientation substantially corresponding to the second configuration of the spinal correction template. In one embodiment, the spinal correction template can be formed of a shape-memory alloy such as Nitinol. The step of activating occurs at a temperature in the range of about 28 °C to about 37 °C. Hence, the template can have an austenite finish temperature (A_{f}) that is below body temperature (about 37 °C). A spinal correction method using the template further includes attaching a primary spinal rod to at least a portion of a spine after the spine achieves a corrected orientation, and removing the spinal correction template. A secondary spinal rod may be inserted in place of the spinal correction template. In some embodiments, the spinal correction template can remain in the patient's body.

Further, US 5,290,289 discloses a method and implant for the surgical treatment of scoliosis involving a segmental fixation of a rigid rod to the abnormally curved portion of the spine. To this end, the document suggest constructing the rod of a shape-memory alloy, such as nitinol, selected such as to be heated and contoured into an ideal shape for a given patient in the parent phase of the alloy (austenite configuration) prior to implementation. At the time of surgery, the cooled rod in a martensite configuration is deformed to accommodate the existing curvature of the patient's spine and segmentally fixed thereto. The rod is then inductively heated to the transition temperature to effect shape recovery and thereby apply corrective forces to the spine. Specifically, US 5,290,289 discloses the transition temperature to be selected in a 10°C range of normal body temperature.

It is the object of the invention to provide a template for use in manufacturing an implant for spinal or other orthopaedic fixation that can be bent in the OR at about room temperature and that is reusable several times. Further, a method of manufacturing such a template and a method of manufacturing an implant with such a template shall be provided.

The object is solved by a template according to claim 1, a method for manufacturing the template according to claim 12 and a method for producing an implant using the template according to claim 13. Further developments are given in the dependent claims.

The template is configured to change its configuration between a first configuration, in which template is capable of bending in response to a force and is capable for retaining a bent shape, and a second configuration in which the template assumes a memorized shape in response to directing the temperature to a recovery level, and wherein the recovery level of the temperature is above body temperature. By body temperature, a temperature of around 37°C is meant. Hence, the template is in the first configuration during its intended use of bending it in the OR outside or inside a patient's body.

With such a template, the curvature of the section of the spine that has to be corrected or the shape of a bone plate which is intended to bridge bone parts or bones to be immobilized can be reproduced by bending or contouring the template with low force in the OR, either manually or using a tool. The bending or contouring the implant can be performed before or while surgery takes place. Afterwards the implant can be manufactured by duplicating the shape of the template using another material for the implant and applying higher forces, usually with a tool.

With the template, difficult shapes can be easily reproduced and an improved anatomical contour can be obtained.

The memorized shape of the template, such as a straight or even shape, can be easily achieved by heating the template to a temperature above the recovery level so that the template automatically assumes the memorized shape. A step of bending back is no longer necessary. After recovery of the memorized shape the template may be re-used for another procedure. The transformation from the first configuration to the second configuration and vice-versa is reversible. Therefore, the template can be re-used a great, even an infinite number of times. An active cooling step is not needed to achieve the first configuration. If the template is not kept at a temperature above the recovery level, the temperature of the template automatically decreases so that the template assumes the first configuration in which it is deformable.

Further, it is possible to revise the formed shape of the template through mild heating to a temperature below the recovery level.

The template may be, for example, a spinal rod or a bone plate. The rod template may be used for producing a spinal rod for correcting a deformity or misalignment in the spinal column caused by disorder such as, for example, scoliosis or caused by injuries. Templates for producing bone plates with a bent shape may be used, for example, in the case of fractures of the hand or the shoulder.

In one embodiment, the template is made of a nickel-titanium (NiTi) alloy with an austenite finish temperature (A_{f}) which is above body temperature, in particular above 45 °C, preferably above 50 °C, more preferably above 60 C and most preferably about 70 °C to about 80°C. Hence, the second configuration which is the memorized shape, can be easily achieved by sterilizing the template with the usual procedure of sterilizing medical instruments, such as vapour sterilization or by immersing it in hot water. Thereby, the recovery level of the template to achieve the memorized shape is easily achieved. The NiTi alloy is biocompatible, therefore, the template is usable inside and outside the human body.

In one embodiment, the NiTi alloy comprises around 49.0 to 52.0 at.-% (atomic percent) nickel, more preferably 49.5 - 50.0 at.% nickel. The remainder is titanium. Hence, the alloy is a martensitic shape memory alloy that has a martensite finish temperature (M_{f}) that is above room temperature. By room temperature a temperature of around 23°C ±3°C is meant.

The strength, in particular the bending stiffness of the template made of this material is less than that of conventional AlMgSi templates. Hence, the template can be bent with less force.

The template can be manufactured in a simple manner including conventional steps of hot drawing and annealing. Thereafter, the template may be cut to size and may be laser marked and finally cleaned.

During manufacturing, the template may obtain an oxide layer on the surface, in particular during a step of annealing. The oxide layer has a colour that differs from the colour of the surfaces of the implants. Therefore, the template can be easily distinguished from the implants. The oxide layer also allows laser marking and imprinting other markings, such as length scales etc.

In the case of the NiTi template, the detection under X-rays is improved due to the higher density compared to the conventional AlMgSi templates.

Further features and advantages of the invention will become apparent from the description of embodiments by means of the accompanying drawings. In the drawings:
- Fig. 1: shows a schematic view of a first embodiment of the template in a second configuration.
- Fig .2: shows a schematic view of the first embodiment of the template in a first configuration.
- Fig. 3: shows a schematic view of the template of the first embodiment adapted to the desired curvature of the portion of the spinal column to be treated and an implant produced on the basis of the template.
- Fig. 4: shows a schematic perspective view of a second embodiment of the template in a first configuration.

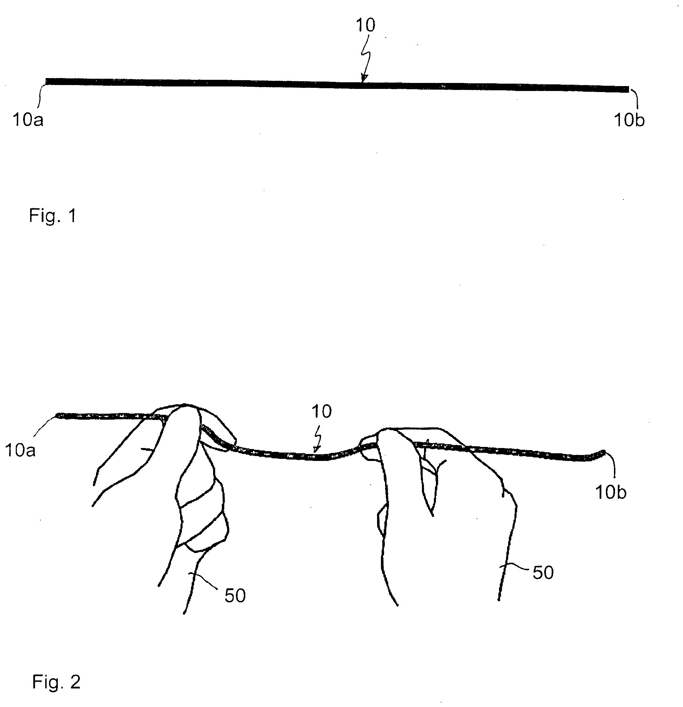

Referring to Figs. 1 and 2, a first embodiment of the template realized by a rod 10. The rod 10 extends between opposite ends 10a, 10b. It may have a cylindrical shape throughout its length and may have the same cross-sectional diameter through its entire length. However, it shall be understood that the rod 10 may have any cross-sectional shape deviating from a cylindrical shape which can be the same or can vary along the length of the rod. The rod 10 can be solid, i.e. without cavities inside or at the surface.

In a second configuration, shown in Fig. 1, the rod 10 assumes a memorized shape. In the embodiment, the memorized shape is a straight shape. However, any other pre-configured memorized shape can be contemplated, such as a shape with a slight curvature between the ends 10a, 10b. The rod 10 is configured to assume the second configuration in response to directing the temperature acting onto the rod 10 to a recovery level. As long as a temperature acting onto the rod 10 is above the recovery level, the rod 10 is configured to maintain the memorized shape.

Fig. 2 shows the rod 10 in a first configuration in which it is capable of bending in response to a force, such as a force exerted by the hands 50 of a user. In the first configuration, the rod 10 is capable of retaining a bent shape when the bending force is no longer applied. The rod 10 is in this first configuration at a temperature level of less than the recovery level, for example at body temperature or less, such as at room temperature.

The rod 10 includes a shape memory material that is characterized by having two distinct configurations and the ability to restore itself, for example by heating it to a temperature above the recovery level, to a memorized shape that has been pre-configured.

More specifically, the material of the rod 10 is a shape memory alloy and still more specifically a nickel-titanium alloy (NiTi alloy) exhibiting the shape memory effect. In the first configuration, the material is in the martensitic metallurgical state where the rod 10 is flexible and deformable and can be caused to assume any of a variety of shapes. Hence, the rod 10 can be bent by exerting a low force, for example manually, onto the rod 10 to cause the rod 10 to assume a bent shape. By directing the temperature above the recovery level, i.e. by heating the rod 10 to a temperature above the recovery level, the material assumes the austenitic metallurgical state wherein the rod 10 is rigid and assumes the memorized shape. The temperature at which the material begins to transform into the austenitic metallurgical state is the austenite start temperature (Aₛ) and the temperature at which the material has fully transformed into the austenitic metallurgical state is the austenite finish temperature (A_{f}). In the case of the NiTi alloy, the recovery level can be defined as the austenite finish temperature (A_{f}).

When the temperature of the rod 10 falls below the temperature of the recovery level, the material reaches the martensite start transformation temperature Mₛ at which temperature the austenitic metallurgical structure begins to transform into the martensitic metallurgical structure. At the martensite finish temperature M_{f} the alloy is completely converted into the martensitic state. When the rod is reheated, the transformation back to the austenitic structure begins at the austenite start temperature Aₛ and is completed at the austenite finish temperature A_{f}. The transition temperatures A_{f}, Aₛ, M_{f} and Mₛ are determined with a method according to ASTM F2082-06 that is a standard test method for determination of the transformation temperatures of nickel-titanium shape memory alloys by bend and free recovery.

In the preferred embodiment, the A_{f} temperature is selected to be above body temperature, for example above 45 °C, preferably above 50 °C, more preferably above 60 °C and most preferably between about 70 °C to about 80°C. An upper limit of the A_{f} temperature may be about 90°C to about 100°C The martensite finish temperature M_{f} is preferably above room temperature. It depends on the A_{f} temperature of the alloy. For example, a template having an A_{f} temperature of about 80°C may have an Aₛ temperature of about 60°C, a Mₛ temperature of about 50°C and a M_{f} temperature of about 40°C.

A particular example for shape memory alloy of the rod 10 according to the first embodiment is a so-called martensitic nickel titanium alloy with a nickel content that is less than that of nickel titanium alloys having superelastic properties, i.e. preferably less than about 50.6 to 51.0 at.-% nickel according to the standard ASTM F 2063. For example, the nickel content of the alloy according to an embodiment 49.0 to about 52.0 at.%- nickel, preferably about 49.5 to about 50.0 at.-% nickel. The remainder is titanium. Small portions of impurities may be present as listed in ASTM F 2063. An example of such an alloy is SM 495. This material can be deformed at room temperature and is transformed back to the austenitic metallurgical state at a temperature A_{f} higher than body temperature.

The rod 10 may have on its surface an oxide layer that is obtained through annealing during a step of manufacturing the rod 10. The oxide layer may have a color that is darker than conventional oxide layers of permanent implants. This results from the fact that the oxide layer obtained through annealing is thicker than conventional oxide layers on implants that may be obtained by anodic oxidation. The oxide layer may represent a marking to distinguish the rod by its color from implants.

On the surface of the rod 10 markings (not shown) may be provided that are obtainable by laser marking. Such markings can include CE marking, LOT-number marking and/or other markings. For example, the rod 10 may have incremental distance markings.

A flexural strength of the rod 10 in the first configuration is, for example, around 100-150 N/mm². The rod may have a diameter corresponding to usual diameters of spinal correction rods, for example, a diameter of 3.5 mm, 4.5 mm, 5.5 mm etc.. The density of the material of the rod may be considerably higher than that of convention AlMgSi rods, for example, around 6 g/cm³ or higher. This causes improved detection through X-rays.

A method of manufacturing the template rod 10 includes steps of forming a pre-configured shape, memorizing the pre-configured shape such that the template has the pre-configured memorized shape in the austenitic metallurgical state. The manufacturing steps include hot drawing and annealing. Typical conditions for annealing are annealing at around 350°C to around 800°C for around 5 minutes to around 60 minutes. Suitable conditions may be selected dependent on the specific composition of the alloy. By the annealing, the oxide layer is obtained. The method may further include steps of cutting to size and/or of laser marking and/or of cleaning the template.

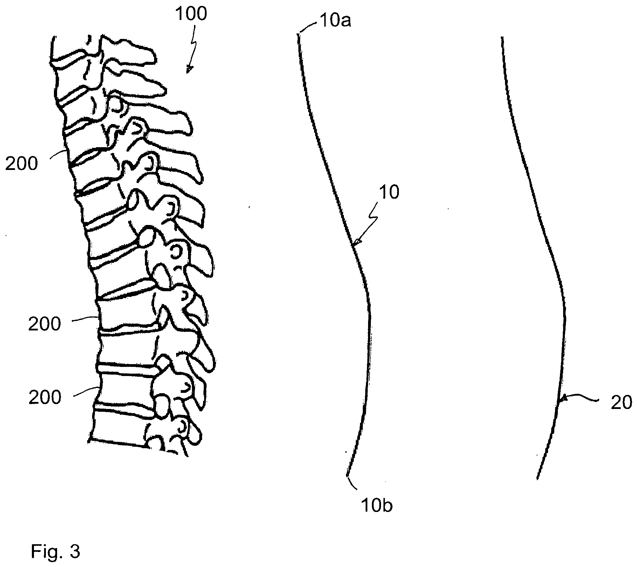

Turning now to Fig. 3, use of the template is shown schematically. The rod 10 is bent in the OR along the actual curvature or the desired curvature of the portion of the spinal column 100 including a number of vertebrae 200. In the OR, there is normally room temperature so that the rod 10 is in the martensitic metallurgical state. It can be easily bent manually or with a device. Alternatively, the rod 10 is inserted into bone anchors (not shown) implanted in the vertebrae to be stabilized. In this case, the bending takes place in the body of the patient. As the recovery level of the temperature is above body temperature (about 37 °C), the rod 10 is still in the martensitic metallurgical state. After the desired curvature is achieved, the rod 10 forms the template for a permanent spinal rod 20 that is bent to have a duplicate shape of the rod 10. Bending of the rod 20 may be performed with a device. The permanent spinal rod 20 is then inserted into the bone anchors (not shown) to stabilize the portion of the spine to be treated.

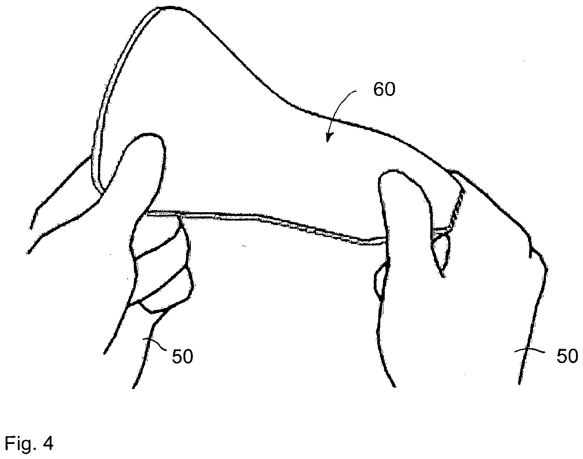

Turning now to Fig. 4, a second embodiment of the template is shown which is in the form of a bone plate 60. The bone plate 60 as shown has an elongate shape, i.e. its greatest length is greater than the greatest width. The thickness of the bone plate 60 is such that the bone plate 60 can be bent in the first configuration as depicted in Fig. 4 by exerting a force with the hands 50. It is not necessary that the thickness of the bone plate 60 is identical to the thickness of a permanent bone plate implant to be manufactured using the template bone plate 60. The bone plate 60 is made of the same material as the rod 10 according to the first embodiment. Hence, the properties of the bone plate 60 with regard to the first configuration and the second configuration are identical. In the second configuration, the bone plate 60 is in the memorized shape, which may be a straight shape, but can also be a slightly bent shape. The contour of the bone plate 60 can be any contour, such as rectangular, irregular, square, oval etc..

The method of manufacturing the bone plate 60 and the method of producing a permanent bone plate implant are the same as described for the first embodiment.

Further modifications of the above described embodiments may be contemplated. For example, instead of spinal rods or bone plates, any other orthopaedic components or implants may be manufactured using a correspondingly shaped template, such as bone nails or pegs or hooks.

Instead of a nickel titanium alloy, other shape memory alloys or shape memory polymers (SMPs) can be used. Such shape memory polymers may include linear block copolymers, for example shape memory polyurethane, thermoplastic polymers, for example polynorbornene, or chemically cross-linked SMPs. The recovery level is then defined as the temperature at which the material begins to transform into the first configuration to reach its memorized shape.

The memorized shape can be any shape. Hence, bending the template and the resulting bent shape of the template in the first configuration includes generating a deviation form the memorized shape.

## Claims

1. A template for use in producing an implant for use in spinal or other orthopaedic fixation,
wherein the template (10, 60) is made of a nickel-titanium shape memory alloy and is configured to change its configuration between a first configuration in which the template (10, 60) is capable of bending in response to a force and is capable of retaining a bent shape and a second configuration in which the template (10, 60) assumes a memorized shape in response to directing the temperature to a recovery level,
**characterized in that** the recovery level of the temperature is the austenite finish temperature (A_{f}) which is above 60°C.

2. The template of claim 1, wherein the shape of the template is a rod (10) or a plate (60).

3. The template of claim 1 or 2, wherein the template (10, 60) is in the first configuration in the martensitic state.

4. The template of one of claims 1 to 3, wherein the recovery level of the temperature is the austenite finish temperature (A_{f}) which is between about 70°C and about 80°C.

5. The template of one of claims 1 to 4, wherein the alloy comprises about 49.0 to 52.0 at.% nickel, preferably about 49.5 to 50.0 at.% nickel.

6. The template of one of claims 1 to 5, wherein the alloy comprises about 48.0 to 51.0 at.% titanium, preferably about 50.0 to 50.5 at.% titanium.

7. The template of one of claims 1 to 6, wherein the template (10, 60) is in the first configuration during room temperature.

8. The template of one of claims 1 to 7, wherein the template (10, 60) is capable to assume its second configuration during a sterilization process, for example vapour sterilization, as used for medical instruments or by immersing it into a hot fluid, such as hot water.

9. The template of one of claims 1 to 8, wherein the template (10, 60) is capable to assume the first configuration upon directing the temperature below the recovery level and wherein the transformation from the first configuration to the second configuration and vice versa can be made at least two, preferably a plurality of times.

10. The template of one of claims 1 to 9, wherein the template (10, 60) comprises a surface with a colour different from the colour of the surface of the implant to be produced, preferably a surface formed by an oxide layer.

11. The template of one of claims 1 to 10, wherein the memorized shape of the template (10, 60) is a straight shape.

12. A method of manufacturing a template according to one of claims 1 to 11, the method comprising the steps of
selecting a material of the template to be a nickel-titanium shape memory alloy whose austenite finish temperature (A_{f}) is above 60°C,
forming a shape of the template (10, 60) by hot drawing,
annealing the template.

13. A method of manufacturing an implant for spinal or other orthopaedic surgery, the method comprising the steps
using a template (10, 60) according to one of claims 1 to 11 in the first configuration after it has been bent;
forming the implant from a semi-finished implant by bending the semi-finished implant corresponding to the bent shape of the template.

## Patentansprüche

1. Schablone zur Verwendung beim Herstellen eines Implantats zur Verwendung bei einer Wirbelsäulenfixierung oder einer anderen orthopädischen Fixierung,
wobei die Schablone (10, 60) aus einer Nickel-Titan-Formgedächtnislegierung ist und dazu ausgebildet ist, ihre Konfiguration zwischen einer ersten Konfiguration, in der die Schablone (10, 60) als Reaktion auf eine Kraft biegefähig ist und zum Behalten einer gebogenen Form fähig ist, und einer zweiten Konfiguration, in der die Schablone (10, 60) als Reaktion auf das Führen der Temperatur zu einem Wiederherstellungslevel eine in ihr Gedächtnis eingeprägte Form annimmt, zu ändern,
**dadurch gekennzeichnet, dass** der Wiederherstellungslevel der Temperatur die Austenit-Enddemperatur (A_{f}) ist, die höher als 60°C ist.

2. Schablone nach Anspruch 1, wobei die Form der Schablone ein Stab (10) oder eine Platte (60) ist.

3. Schablone nach Anspruch 1 oder 2, wobei die Schablone (10, 60) in der ersten Konfiguration im Martensit-Zustand ist.

4. Schablone nach einem der Ansprüche 1 bis 3, wobei der Wiederherstellungslevel der Temperatur die Austenit-Enddemperatur (A_{f}) ist, die zwischen etwa 70°C und etwa 80°C ist.

5. Schablone nach einem der Ansprüche 1 bis 4, wobei die Legierung etwa 49,0 bis 52,0 at.% (Atomprozent) Nickel, vorzugsweise etwa 49,5 bis 50,0 at.% Nickel umfasst.

6. Schablone nach einem der Ansprüche 1 bis 5, wobei die Legierung etwa 48,0 bis 51,0 at.% Titan, vorzugsweise etwa 50,0 bis 50,5 at.% Titan umfasst.

7. Schablone nach einem der Ansprüche 1 bis 6, wobei die Schablone (10, 60) bei Raumtemperatur in der ersten Konfiguration ist.

8. Schablone nach einem der Ansprüche 1 bis 7, wobei die Schablone (10, 60) dazu fähig ist, ihre zweite Konfiguration bei einem Sterilisationsvorgang, beispielsweise einer Dampfsterilisation, wie für medizinische Instrumente verwendet, oder durch das Eintauchen der Schablone in ein heißes Fluid, wie z. B. heißes Wasser, anzunehmen.

9. Schablone nach einem der Ansprüche 1 bis 8, wobei die Schablone (10, 60) dazu fähig ist, die erste Konfiguration nach dem Senken der Temperatur unter den Wiederherstellungslevel anzunehmen, und wobei die Umwandlung von der ersten Konfiguration zu der zweiten Konfiguration und umgekehrt mindestens zweimal, vorzugsweise mehrmals, durchgeführt werden kann.

10. Schablone nach einem der Ansprüche 1 bis 9, wobei die Schablone (10, 60) eine Oberfläche mit einer anderen Farbe als die Farbe der Oberfläche des herzustellenden Implantats, vorzugsweise eine durch eine Oxidschicht gebildete Oberfläche, umfasst.

11. Schablone nach einem der Ansprüche 1 bis 10, wobei die in ihr Gedächtnis eingeprägte Form der Schablone (10, 60) eine gerade Form ist.

12. Verfahren zum Herstellen einer Schablone nach einem der Ansprüche 1 bis 11, wobei das Verfahren folgende Schritte umfasst:
Auswählen einer Nickel-Titan-Formgedächtnislegierung, deren Austenit-Enddemperatur (A_{f}) höher als 60°C ist, als Material der Schablone,
Ausbilden einer Form der Schablone (10, 60) durch das Warmziehen,
Ausglühen der Schablone.

13. Verfahren zum Herstellen eines Implantats für die Wirbelsäulenchirurgie oder andere orthopädische Chirurgie, wobei das Verfahren folgende Schritte umfasst:
Verwenden einer Schablone (10, 60) nach einem der Ansprüche 1 bis 11 in der ersten Konfiguration, nachdem die Schablone gebogen worden ist;
Ausbilden des Implantats aus einem halbfertigen Implantat durch das Biegen des halbfertigen Implantats entsprechend der gebogenen Form der Schablone.

## Revendications

1. Gabarit destiné à être utilisé dans la production d'un implant conçu pour être utilisé dans le cadre de la fixation de la colonne vertébrale ou de toute autre fixation orthopédique,
dans lequel le gabarit (10, 60) est constitué d'un alliage nickel-titane à mémoire de forme et est conçu pour changer sa configuration entre une première configuration, selon laquelle le gabarit (10, 60) est capable de se courber en réponse à une force et est capable de conserver une forme courbée, et une seconde configuration, selon laquelle le gabarit (10, 60) adopte une forme mémorisée en réponse à l'affectation de la température à un niveau de récupération,
**caractérisé en ce que** le niveau de récupération de la température est la température de finition austénite (A_{f}) qui est supérieure à 60°C.

2. Gabarit selon la revendication 1, dans lequel la forme du gabarit est une tige (10) ou une plaque (60).

3. Gabarit selon la revendication 1 ou 2, dans lequel le gabarit (10, 60) est dans la première configuration à l'état martensitique.

4. Gabarit selon l'une des revendications 1 à 3, dans lequel le niveau de récupération de la température est la température de finition austénite (A_{f}) qui se situe entre environ 70°C et environ 80°C.

5. Gabarit selon l'une des revendications 1 à 4, dans lequel l'alliage contient environ 49.0 à 52.0 % atomique de nickel, de préférence environ 49.5 à 50.0 % atomique de nickel.

6. Gabarit selon l'une des revendications 1 à 5, dans lequel l'alliage contient environ 48.0 à 51.0 % atomique de titane, de préférence environ 50.0 à 50.5 % atomique de titane.

7. Gabarit selon l'une des revendications 1 à 6, dans lequel le gabarit (10, 60) est dans la première configuration à température ambiante.

8. Gabarit selon l'une des revendications 1 à 7, dans lequel le gabarit (10, 60) est capable d'adopter sa seconde configuration pendant un procédé de stérilisation, par exemple de stérilisation à la vapeur, comme utilisé pour des instruments médicaux ou par immersion dans un liquide chaud, comme de l'eau chaude.

9. Gabarit selon l'une des revendications 1 à 8, dans lequel le gabarit (10, 60) est capable d'adopter la première configuration par l'affectation de la température sous le niveau de récupération, et dans lequel la transformation de la première configuration en la seconde configuration et vice-versa peut être effectuée au moins deux fois, de préférence une multitude de fois.

10. Gabarit selon l'une des revendications 1 à 9, dans lequel le gabarit (10, 60) comprend une surface avec une couleur différente de la couleur de la surface de l'implant à produire, de préférence une surface formée par une couche d'oxyde.

11. Gabarit selon l'une des revendications 1 à 10, dans lequel la forme mémorisée du gabarit (10, 60) est une forme droite.

12. Procédé de fabrication d'un gabarit selon l'une des revendications 1 à 11, le procédé comprenant les étapes de
sélection d'un matériau du gabarit comme étant un alliage nickel-titane à mémoire de forme, dont la température de finition austénite (A_{f}) est supérieure à 60°C,
mise en forme du gabarit (10, 60)) par étirage à chaud, recuit du gabarit.

13. Procédé de fabrication d'un implant pour chirurgie spinale ou autre chirurgie orthopédique, le procédé comprenant les étapes de
l'utilisation d'un gabarit (10, 60) selon l'une des revendications 1 à 11 dans la première configuration après qu'il ait été courbé ;
mise en forme de l'implant à partir d'un implant semi-fini en courbant l'implant semi-fini en correspondance avec la forme courbée du gabarit.
